# EUROPEAN PATENT APPLICATION

(11) **EP 4 606 817 A1**
(43) Date of publication of application: **27.08.2025**
(21) Application number: 23879958.9
(22) Date of filing: 26.07.2023
(51) Int. Cl.: C07K 14/28, A61K 39/39, A61K 38/00, A61K 39/00

(54) **MANUFACTURE AND APPLICATION OF FLAGELLIN IMMUNOPOTENTIATING DERIVATIVES WITH TLR5 ACTIVITY USING EUKARYOTIC CELL EXPRESSION SYSTEMS**

(30) Priority: 19.10.2022 KR 20220134839
(71) Applicant: Industry Foundation of Chonnam National University, Gwangju 61186 (KR)
(72) Inventor: RHEE, Joon Haeng, Gwangju 61903 (KR); LEE, Shee Eun, Gwangju 61210 (KR); KHIM, Koemchhoy, Hwasun-gun, Jeollanam-do 58116 (KR)
(74) Representative: Plougmann Vingtoft a/s
(86) International application number: PCT/KR2023/010821
(87) International publication number: WO 2024/085375

(57) **Abstract**

The present invention relates to the manufacture and application of flagellin immunopotentiating derivatives with TLR5 activity using an eukaryotic cell expression system. The flagellin derivative according to the present invention has a high immunostimulatory effect and can be used in various vaccine and immunotherapy compositions.

## Description

### Technical Field

The present invention was made with the support of the Ministry of Science and ICT of the Republic of Korea, under Project No. 2020R1A5A2031185, which was conducted (contribution rate: 60%) in the research project named "Combinatorial Tumor Immunotherapy Research Center" in the research program titled "Collective Research Support", by Chonnam National University, under the management of the National Research Foundation of Korea, from 01 June 2018 to 28 February 2025.

The present invention was also made with the support of the Ministry of Science and ICT of the Republic of Korea, under Project No. 2020M3A9G3080282, which was conducted (contribution rate: 20%) in the research project named "Research group for innovative projects" in the research program titled "Biomedical Technology Development", by Chonnam National University, under the management of the National Research Foundation of Korea, from 01 June 2020 to 31 December 2024.

The present invention was also made with the support of the Ministry of Health and Welfare of the Republic of Korea, under Project No. HV22C0079, which was conducted (contribution rate: 20%) in the research project named "Development of a mucosal vaccine adjuvant platform efficiently inducing cellular immune responses" in the research program titled "Vaccine-Based Technology Development", by Chonnam National University, under the management of the National Research Foundation of Korea, from 01 April 2022 to 31 December 2024.

The present invention relates to the manufacture and application of a flagellin adjuvant derivative with TLR5 activity using a eukaryotic cell expression system and, more specifically, to a flagellin protein that can be expressed in a eukaryotic cell and exhibit an immunopotentiating effect equivalent to or higher than that of an existing prokaryote-derived flagellin protein.

### Background Art

The structural unit protein constituting the filaments of bacterial flagella is called flagellin, and flagellin molecules are regularly assembled to form the filament, thereby enabling bacteria to move.

TLR5 is a pattern recognition receptor that is expressed on the surface of various cells including macrophages and dendritic cells. Upon the recognition of flagellin, TLR5 induces NF-κB activation and inflammatory cytokine production. NF-κB, which is a transcription factor that regulates the expression of genes involved in immune and inflammatory responses, contains an inhibitory protein called IκB (inhibitor of NF-κB) and exists as a protein complex in the cytoplasm. TLR5, when activated by stimulation, degrades IκB and induces the activation of NF-κB, and the activation of NF-kB induces the production of pro-inflammatory cytokines, such as tumor necrosis factor-a (TNF-α) and interleukin-1f (IL-1β).

In addition, flagellin is a pathogen-associated molecular pattern that can stimulate the NLRC4-inflammasome signaling system present in the cytoplasm. Therefore, flagellin is an immunomodulatory substance capable of activating both TLR5 existing on the cell surface and the NLRC4-inflammasome present in the cytoplasm.

Through the utilization of these characteristics, flagellin has been studied as a target for the development of adjuvants applicable to various vaccines and immunotherapies. The co-administration of various antigens with flagellin or the administration of flagellin in the form of a recombinant fusion protein enhances antigen-specific immune responses, and the adjuvant efficacy has been validated in influenza vaccines, pneumococcal vaccines, bacterial periodontal disease, cancer therapeutic vaccines, norovirus vaccines, neurodegenerative diseases, and various immune diseases. It has been also reported that the activation of TLR5 by flagellin protects hematopoietic cells and gastrointestinal tissues from radiation and affects the survival and growth of cancer cells.

Until now, the flagellin adjuvant effects have been obtained using recombinant proteins expressed in *E. coli,* which is a prokaryotic cell. However, in order to apply flagellin adjuvants to nucleic acid-based vaccines (e.g., DNA vaccines, mRNA vaccines, etc.) or antigens requiring post-translational modifications, the manufacture of flagellin expressed in eukaryotic expression systems is needed. However, flagellin manufactured in the eukaryotic cell systems shows a significant reduction in TLR5 signaling activity compared with prokaryotic-expressed flagellin. This is presumed to be due to the fact that TLR5-signaling cannot be efficiently induced because of stearic hindrance caused by post-translational modifications during the expression of flagellin in eukaryotic cells.

Therefore, there is a need for research on flagellin proteins that can be manufactured in eukaryotic cells while retaining potent immunomodulatory ability and effective vaccine adjuvant functions.

### Disclosure of Invention

### Technical Problem

Therefore, the present inventors manufactured flagellin proteins in eukaryotic cells and identified significantly excellent immunopotentiating ability and safety of the flagellin proteins.

Specifically, the present inventors identified that flagellin manufactured in eukaryotic cell systems showed very weak TLR5 stimulating activity. This is presumed to be due to the fact that the expression of flagellin in eukaryotic cells results in the manufacture of glycosylated flagellin (glycosylated-eFlaB) by post-translational modifications, and the glycosylated flagellin fails to efficiently induce TLR5-signaling due to the stearic hindrance by glucose molecules. To prove the hypothesis, bioinformatics analysis was conducted, and it was identified that flagellin expressed in eukaryotic cells had five N-glycosylation sites. Thus, flagellin variants in which an asparagine residue presumed as an N-glycosylation site was substituted with alanine were manufactured by introducing site-directed mutagenesis, and the activity of the corresponding flagellin variants was verified in vitro and in vivo conditions. As a result, eukaryotic-expressed flagellin adjuvant variants (eukFlaB variants) exhibiting TLR5 stimulating activity and adjuvant efficacy identical to those of pFlaB were induced. Interestingly, the corresponding variants (eukFlaB variants) exhibited deimmunization. Overall, a variety of safer and more clinically applicable flagellin adjuvants were induced by establishing N-deglycosylated eukaryotic-expressed flagellin proteins through bioinformatic analysis and protein modification strategies.

Accordingly, an aspect of the present invention is to provide a flagellin protein expressible in eukaryotic cells.

Another aspect of the present invention is to provide a vaccine adjuvant composition containing a flagellin protein expressible in eukaryotic cells.

Yet another aspect of the present invention is to provide a composition for immune function enhancement, the composition containing a flagellin protein expressible in eukaryotic cells.

Still yet another aspect of the present invention is to provide a pharmaceutical composition for prevention or treatment of cancer or a microbial infection, the pharmaceutical composition containing a flagellin protein expressible in eukaryotic cells.

Still yet another aspect of the present invention is to provide use of a flagellin protein, expressible in eukaryotic cells, for adjuvant application.

Still yet another aspect of the present invention is to provide use of a flagellin protein, expressible in eukaryotic cells, for immune function enhancement.

Still yet another aspect of the present invention is to provide use of a flagellin protein, expressible in eukaryotic cells, for the prevention or treatment of an autoimmune disease, an inflammatory disease, a microbial infection, or cancer.

### Solution to Problem

The present invention relates to the manufacture and application of a flagellin adjuvant derivative with TLR5 activity using a eukaryotic cell expression system, and the flagellin protein according to the present invention exhibits a high immunopotentiating effect.

Hereinafter, the present invention will be described in more detail.

In accordance with an aspect of the present invention, a polypeptide expressible in a eukaryotic cell is provided in which at least one asparagine (N) residue in the amino acid sequence of SEQ ID NO: 13 is substituted with alanine.

In an embodiment of the present invention, in the polypeptide, an asparagine residue at at least one position selected from the group consisting of positions 83, 101, 139, 273, and 330 in the amino acid sequence of SEQ ID NO: 13 is substituted with alanine.

In an embodiment of the present invention, in the polypeptide, asparagine residues at position 83 and at least one position selected from the group consisting of positions 101, 139, 273, and 330 in the amino acid sequence of SEQ ID NO: 13 are substituted with alanine.

In an embodiment of the present invention, in the polypeptide, asparagine residues at positions 83 and 101 and at least one position selected from the group consisting of positions 139, 273, and 330 in the amino acid sequence of SEQ ID NO: 13 are substituted with alanine.

In an embodiment of the present invention, in the polypeptide, asparagine residues at positions 83, 101, 139 and at least one position selected from the group consisting of positions 273 and 330 in the amino acid sequence of SEQ ID NO: 13 are substituted with alanine.

In an embodiment of the present invention, the polypeptide may include one amino acid sequence selected from the group consisting of amino acid sequences of SEQ ID NOS: 14 to 23.

The polypeptide according to the present invention can exhibit an immunopotentiating effect due to excellent TLR5 activity, and thus can be used as an adjuvant composition for a vaccine, a composition for immune function enhancement, or a pharmaceutical composition for the prevention or treatment of an autoimmune disease, an inflammatory disease, a microbial infection, or cancer, but is not limited thereto.

The polypeptide according to the present invention exhibits an excellent effect in inducing the production of antibodies specific to influenza antigens when administered in combination with influenza vaccines, and can be therefore used as an adjuvant composition for an influenza vaccine or an adjuvant composition for influenza treatment, but is not limited thereto.

In accordance with another aspect of the present invention, an adjuvant composition for a vaccine is provided, the composition containing a polypeptide in which at least one asparagine (N) residue in the amino acid sequence of SEQ ID NO: 13 is substituted with alanine.

In an embodiment of the present invention, in the polypeptide, an asparagine residue at at least one position selected from the group consisting of positions 83, 101, 139, 273, and 330 in the amino acid sequence of SEQ ID NO: 13 may be substituted with alanine.

In an embodiment of the present invention, in the polypeptide, asparagine residues at position 83 and at least one position selected from the group consisting of positions 101, 139, 273, and 330 in the amino acid sequence of SEQ ID NO: 13 may be substituted with alanine.

In an embodiment of the present invention, in the polypeptide, asparagine residues at positions 83 and 101 and at least one position selected from the group consisting of positions 139, 273, and 330 in the amino acid sequence of SEQ ID NO: 13 may be substituted with alanine.

In an embodiment of the present invention, in the polypeptide, asparagine residues at positions 83, 101, and 139 and at least one position selected from the group consisting of positions 273 and 330 in the amino acid sequence of SEQ ID NO: 13 may be substituted with alanine.

In an embodiment of the present invention, the polypeptide may include one amino acid sequence selected from the group consisting of amino acid sequences of SEQ ID NOS: 14 to 23.

The adjuvant composition for a vaccine according to the present invention may further contain at least one commonly used pharmaceutically and/or veterinarily acceptable carrier, diluent, antibiotic, or adjuvant.

The carrier may include any solvent (solution), dispersion medium, coating agent, buffer, isotonic agent, suspension, colloid, stabilizer, preservative, antibiotic agent (antibacterial agent, antifungal agent, etc.), adsorption retardant, auxiliary agent (adjuvant), implant, or the like, which is pharmaceutically acceptable for administration to a target animal (host animal). In general, the use of at least one vehicle for a chemical compound, especially, an immunogen, is well known to a person skilled in the pharmaceutical industry. Any conventional medium or agent is considered to be used in the diagnostic, prophylactic, or therapeutic composition as long as the medium or agent is not compatible with an active ingredient. One or more supplementary active ingredients may also be incorporated into at least one of the disclosed immunogenic composition or vaccine formulation, or may be administered in combination therewith.

Examples of the diluent may include water, a saline solution, dextrose, ethanol, glycerol, and the like, examples of the isotonic agent may include sodium chloride, dextrose, mannitol, sorbitol, lactose, and the like, and examples of the stabilizer may include albumin, but are not limited thereto.

Examples of the auxiliary agent (adjuvant) may include: mineral gels, such as aluminum hydroxide gel; surfactants, such as lysolecithin; glycosides, such as saponin derivatives, for example, Quil A or GPI-0100 (Galenica Pharmaceuticals, Inc., Birmingham, Alabama); pluronic polyols; multivalent anions (polyanions); nonionic block copolymers, such as Pluronic F-127 (B.A.S.F., USA); peptides; mineral oils, such as Montanide ISA-206 (Seppic, France), Montanide ISA-50 (Seppic, France), Carbopol, Amphigen, Amphigen Mark II (Hydronics, USA), Alhydrogel; oil emulsions (e.g., emulsions of mineral oil and water, such as BayolF/Arlacel A) or emulsions of vegetable oil, water, and an emulsifier such as lecithin; alum; cholesterol; Freund's complete and incomplete adjuvants; block copolymers (CytRx, Atlanta, Georgia); SAF-M (Chiron, Emeryville, California); AMPHIGEN^{®} adjuvants; monophosphoryl lipid A; avridine lipid-amine adjuvants; *E.* coli-derived heat-labile enterotoxins (recombination or the like); cholera toxins; muramyl dipeptides; IMS1313 (Seppic, France); ISA206; Montanide 01 gel (Seppic, France); or mixtures of these adjuvants, but are not limited thereto. Preferably, the adjuvant is at least one selected from the group consisting of IMS1313, Carbopol, and Montanide 01 gel. The adjuvant refers to a compound or mixture that enhances the immune response and/or promotes the absorption rate after inoculation, and may include any absorption promoting agent.

Still another aspect of the present invention is directed to a composition for immune function enhancement, the composition containing a polypeptide in which at least one asparagine (N) residue in the amino acid sequence of SEQ ID NO: 13 is substituted with alanine.

In an embodiment of the present invention, in the polypeptide, an asparagine residue at at least one position selected from the group consisting of positions 83, 101, 139, 273, and 330 in the amino acid sequence of SEQ ID NO: 13 may be substituted with alanine.

In an embodiment of the present invention, in the polypeptide, asparagine residues at position 83 and at least one position selected from the group consisting of positions 101, 139, 273, and 330 in the amino acid sequence of SEQ ID NO: 13 may be substituted with alanine.

In an embodiment of the present invention, in the polypeptide, asparagine residues at positions 83 and 101 and at least one position selected from the group consisting of positions 139, 273, and 330 in the amino acid sequence of SEQ ID NO: 13 may be substituted with alanine.

In an embodiment of the present invention, in the polypeptide, asparagine residues at positions 83, 101, and 139 and at least one position selected from the group consisting of positions 273 and 330 in the amino acid sequence of SEQ ID NO: 13 may be substituted with alanine.

In an embodiment of the present invention, the polypeptide may include one amino acid sequence selected from the group consisting of amino acid sequences of SEQ ID NOS: 14 to 23.

Still another aspect of the present invention is directed to a pharmaceutical composition for the prevention or treatment of an autoimmune disease, an inflammatory disease, a microbial infection, or cancer, the pharmaceutical composition containing a polypeptide in which at least one asparagine (N) residue in the amino acid sequence of SEQ ID NO: 13 is substituted with alanine.

In an embodiment of the present invention, the autoimmune disease may be at least one selected from the group consisting of hemophagocytic lymphohistiocytosis, systemic lupus erythematosus, Kikuchi disease, vasculitis, adult-onset Still's disease, rheumatoid arthritis, inflammatory myositis, Behçet's disease, IgG4-related disease, Sjögren's syndrome, giant cell arteritis, temporal arteritis, type 1 diabetes, atopic dermatitis, Crohn's disease, systemic sclerosis, psoriasis, multiple sclerosis, and Graves' hyperthyroidism, but is not limited thereto.

In an embodiment of the present invention, the inflammatory disease may be at least one selected from the group consisting of sepsis, gastritis, enteritis, nephritis, hepatitis, chronic obstructive pulmonary disease (COPD), pulmonary fibrosis, irritable bowel syndrome, inflammatory pain, migraine, headache, backache, fibromyalgia, myofascial disorders, viral infections, bacterial infections, fungal infections, burns, wounds from surgical or dental procedures, prostaglandin excess syndrome, atherosclerosis, gout, Hodgkin's disease, pancreatitis, conjunctivitis, iritis, scleritis, uveitis, and eczema, but is not limited thereto.

In an embodiment of the present invention, the microorganism may be at least one selected from the group consisting of Influenza virus, Corona virus, Hepatitis virus, Zika virus, Dengue virus (DENV), Hanta virus, Cytomegalovirus (CMV), Epstein Barr virus (EBV), human immunodeficiency virus (HIV), Herpes simplex virus (HSV), Chikungunya virus, Enterovirus, Marburg hemorrhagic fever virus, parvovirus, Sever Fever with Thrombocytopenia Syndrome Virus (SFTSV), Rotavirus, Norovirus, *Mycobacterium tuberculosis,* Group A *Streptococcus* (GAS), Group B *Streptococcus* (GBS), *Staphylococcus aureus, Shigella,* pathogenic *E. coli, Salmonella, Chlamydia, Pseudomonas aeruginosa,* Non-typeable *Haemophilus influenzae, Klebsiella pneumoniae, Clostridium difficile, Plasmodium, Leishmania, Schistosoma, Trypanosoma, Brucella, Cryptosporidium* and *Entamoeba,* but is not limited thereto.

In an embodiment of the present invention, the cancer may be at least one selected from the group consisting of Breast cancer, lung cancer, colon cancer, kidney cancer, liver cancer, ovarian cancer, prostate cancer, testicular cancer, urogenital cancer, lymphatic cancer, rectal cancer, pancreatic cancer, esophageal cancer, stomach cancer, cervical cancer, thyroid cancer, and skin cancer, but is not limited thereto.

In an embodiment of the present invention, in the polypeptide, an asparagine residue at at least one position selected from the group consisting of positions 83, 101, 139, 273, and 330 in the amino acid sequence of SEQ ID NO: 13 may be substituted with alanine.

In an embodiment of the present invention, in the polypeptide, asparagine residues at position 83 and at least one position selected from the group consisting of positions 101, 139, 273, and 330 in the amino acid sequence of SEQ ID NO: 13 may be substituted with alanine.

In an embodiment of the present invention, in the polypeptide, asparagine residues at positions 83 and 101 and at least one position selected from the group consisting of positions 139, 273, and 330 in the amino acid sequence of SEQ ID NO: 13 may be substituted with alanine.

In an embodiment of the present invention, in the polypeptide, asparagine residues at positions 83, 101, and 139 and at least one position selected from the group consisting of positions 273 and 330 in the amino acid sequence of SEQ ID NO: 13 may be substituted with alanine.

In an embodiment of the present invention, the polypeptide may include one amino acid sequence selected from the group consisting of amino acid sequences of SEQ ID NOS: 14 to 23.

The pharmaceutical composition of the present invention may be administered at a pharmaceutically effective amount.

As used herein, the term "pharmaceutically effective amount" refers to an amount that is sufficient to attain the efficacy or activity of the above-described composition.

The pharmaceutical composition of the present invention may contain a pharmaceutically acceptable carrier.

The pharmaceutically acceptable carrier contained in the pharmaceutical composition of the present invention is commonly used in the formulation of medicines, and may include lactose, dextrose, sucrose, sorbitol, mannitol, starch, gum acacia, calcium phosphate, alginates, gelatin, calcium silicate, micro-crystalline cellulose, polyvinylpyrrolidone, cellulose, water, syrup, methyl cellulose, methylhydroxy benzoate, propylhydroxy benzoate, talc, magnesium stearate, and mineral oil, but is not limited thereto. The pharmaceutical composition of the present invention may further contain, in addition to the above ingredients, a lubricant, a wetting agent, a sweetening agent, a flavoring agent, an emulsifier, a suspending agent, a preservative, and the like.

The pharmaceutical composition according to the present invention may be administered to mammals including humans through various routes. The manner of administration may be any manner that is conventionally used, and the administration may be conducted through an oral, dermal, intravenous, intramuscular, or subcutaneous route, and for example, the administration may be conducted through an oral route.

The appropriate dose of the pharmaceutical composition of the present invention varies depending on factors, such as a formulating method, a manner of administration, patient's age, body weight, sex, and morbidity, food, a time of administration, a route of administration, an excretion rate, and response sensitivity. An ordinarily skilled practitioner can easily determine and prescribe a dose effective for the desired treatment or prevention.

The pharmaceutical composition of the present invention may be formulated using a pharmaceutically acceptable carrier and/or vehicle by a method that can be easily performed by a person having ordinary skills in the art to which the present invention pertains, so that the pharmaceutical composition can be prepared in a unit dose form or can be contained in a multi-dose container. The formulation may be in the form of a solution in an oily or aqueous medium, a suspension, an emulsion, an extract, a powder, granules, a tablet, a capsule, or a gel (e.g., a hydrogel), and may further contain a dispersant or a stabilizer.

Still another aspect of the present invention is directed to use of a polypeptide, in which at least one asparagine (N) residue in the amino acid sequence of SEQ ID NO: 13 is substituted with alanine, for vaccine adjuvant application.

Still another aspect of the present invention is directed to use of a polypeptide, in which at least one asparagine (N) residue in the amino acid sequence of SEQ ID NO: 13 is substituted with alanine, for immune function enhancement.

Still another aspect of the present invention is directed to use of a polypeptide, in which at least one asparagine (N) residue in the amino acid sequence of SEQ ID NO: 13 is substituted with alanine, for the prevention or treatment of an autoimmune disease, an inflammatory disease, a microbial infection, or cancer.

### Advantageous Effects of Invention

The present invention relates to the manufacture and application of a flagellin adjuvant derivative with TLR5 activity using a eukaryotic cell expression system, and the flagellin variant according to the present invention exhibits a high immunopotentiating effect and can be used in various vaccines and immunotherapeutic compositions.

### Brief Description of Drawings

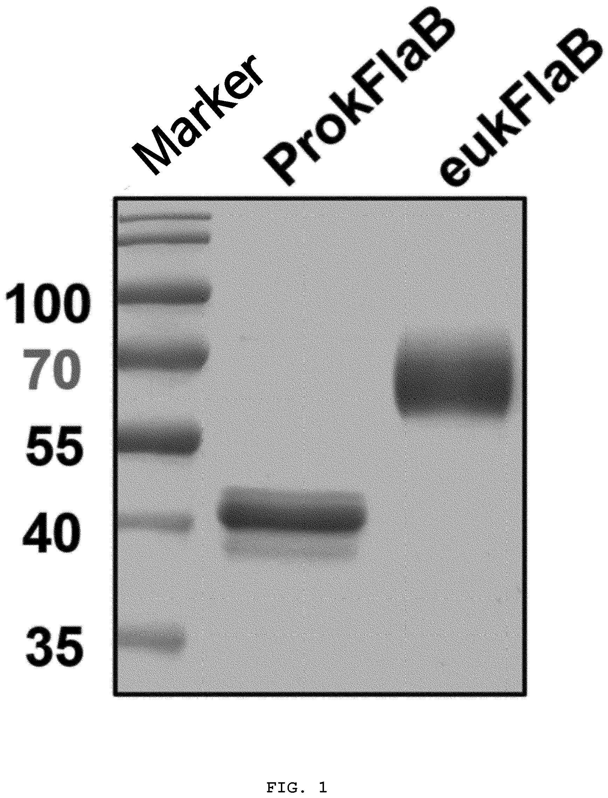

FIG. 1 shows the western blot analysis results of *Vibrio vulnificus-derived* wild-type flagellin (prokFlaB) expressed in *E. coli* cells, which are prokaryotic cells, and wild-type flagellin (eukFlaB) expressed in Expi293^{™} cells, which are eukaryotic cells, according to an example of the present invention.

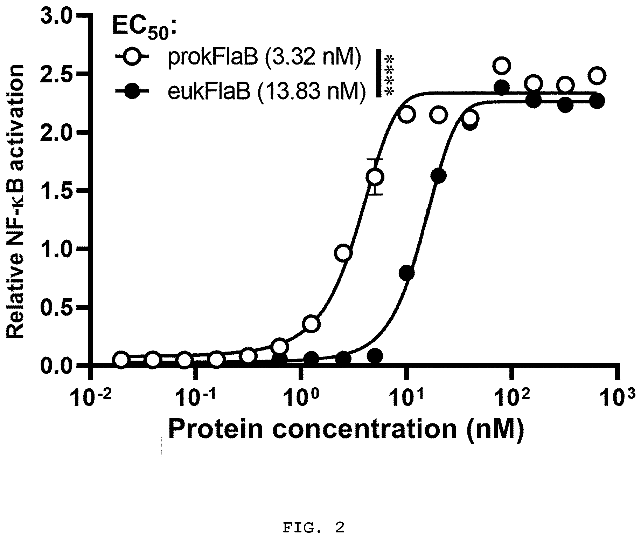

FIG. 2 is a graph showing the comparison results of TLR5 stimulating activity between *Vibrio vulnificus-*derived wild-type flagellin (prokFlaB) expressed in E. *coli* cells, which are prokaryotic cells, and wild-type flagellin (eukFlaB) expressed in Expi293^{™} cells, which are eukaryotic cells, according to an example of the present invention.

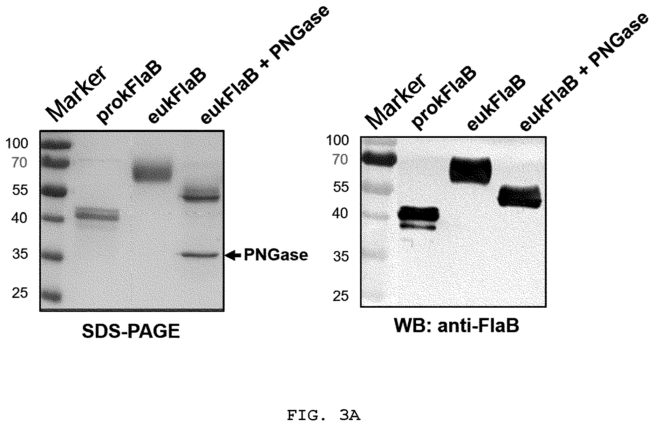

FIG. 3A shows the Western blot analysis results of a protein before and after the wild-type flagellin (eukFlaB) expressed in Expi293^{™} cells, which are eukaryotic cells, was treated with the deglycosylase Peptide N-glycosidase F (PNGase) according to an example of the present invention.

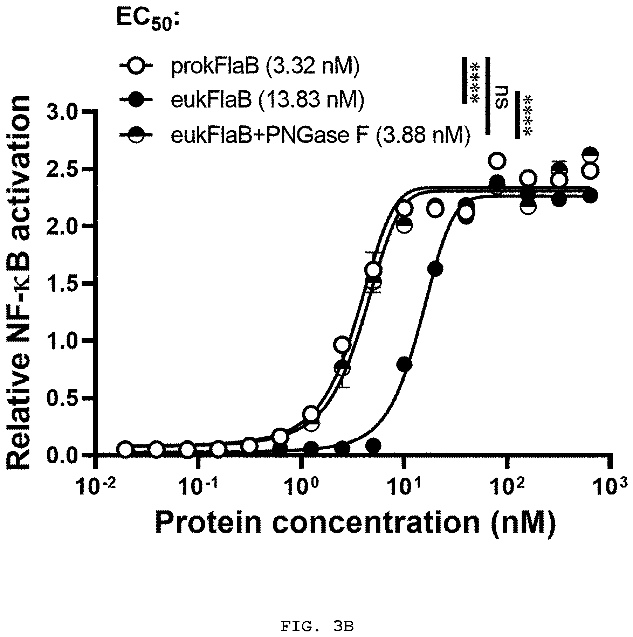

FIG. 3B is a graph showing the comparison results of TLR5 stimulating activity before and after the wild-type flagellin (eukFlaB) expressed in Expi293^{™} cells, which are eukaryotic cells, was treated with the deglycosylase Peptide N-glycosidase F (PNGase) according to an example of the present invention.

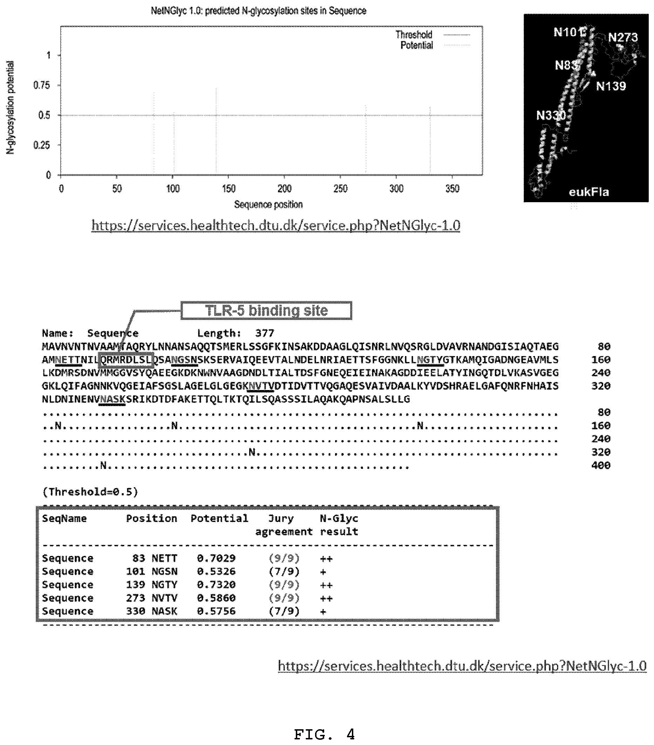

FIG. 4 shows the results of predicting the glycosylated amino acid sequence of flagellin (FlaB) according to an example of the present invention.

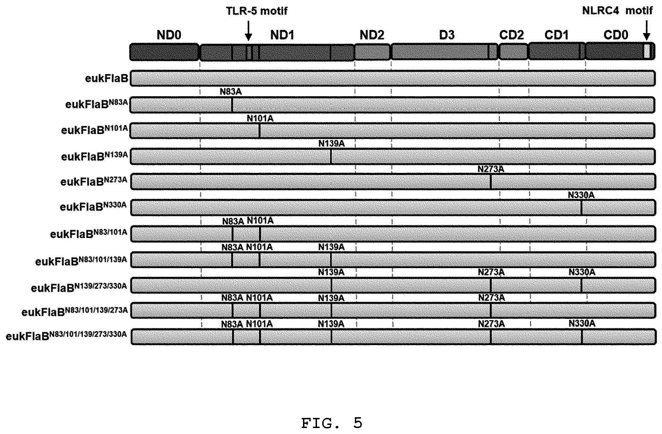

FIG. 5 shows the construction of flagellin variants induced by site-directed mutagenesis.

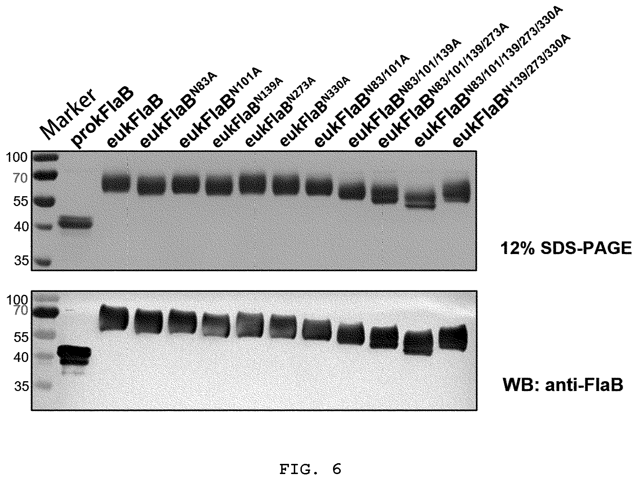

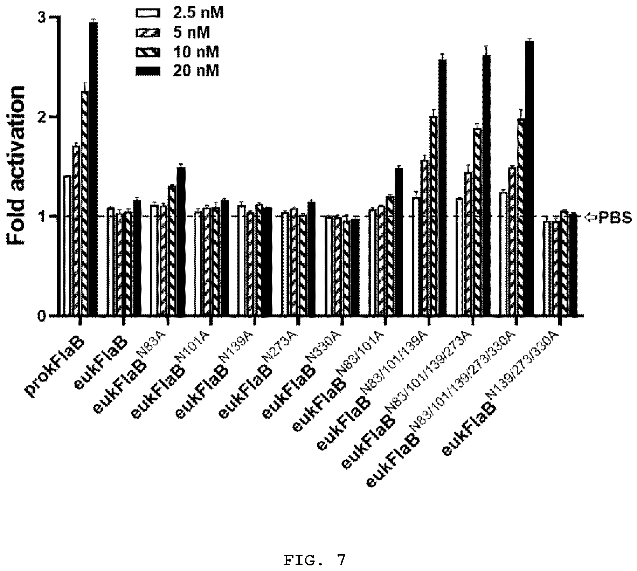

FIGS. 6 and 7 show the results of verifying features of flagellin variants expressed in Expi293^{™} cells, which are eukaryotic cells and TLR5 stimulating activities thereof according to examples of the present invention.

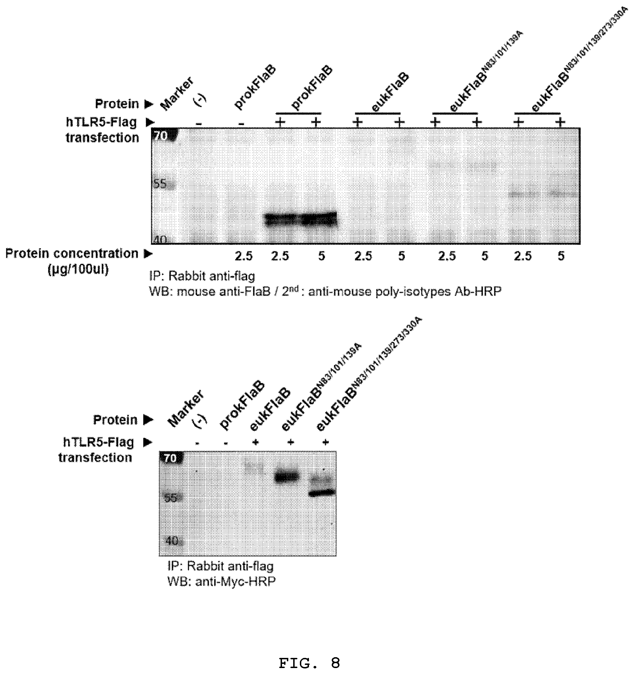

FIG. 8 shows the results of determining TLR5 binding with flagellin variants by co-immunoprecipitation (Co-IP).

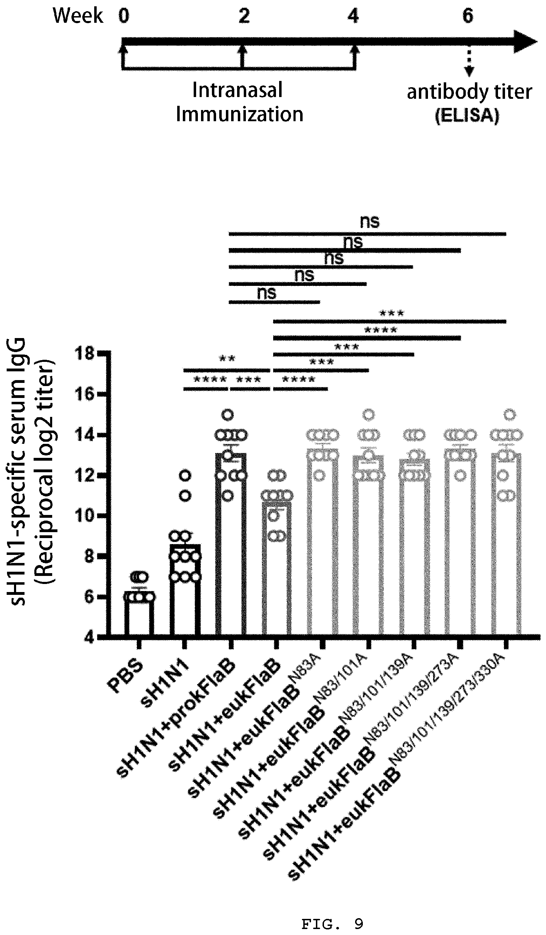

FIG. 9 shows the results of comparing adjuvant efficacy among *Vibrio vulnificus-*derived wild-type flagellin (prokFlaB) expressed in *E. coli* cells, which are prokaryotic cells, wild-type flagellin (eukFlaB) expressed in Expi293^{™} cells, which are eukaryotic cells, and various flagellin variants in the influenza vaccine model according to an example of the present invention.

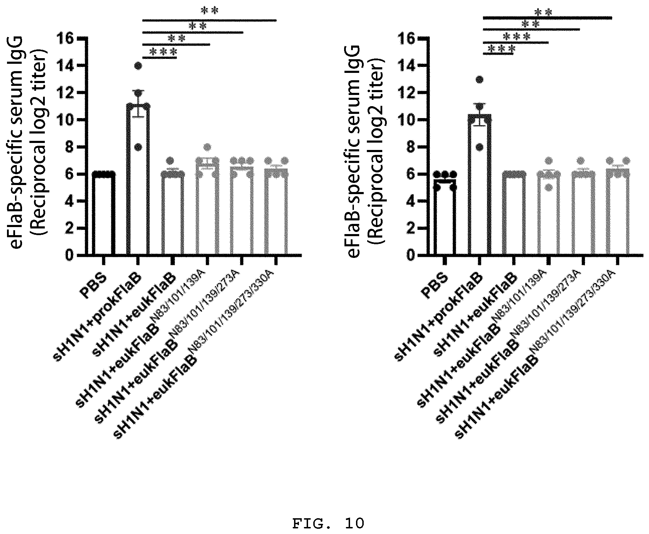

FIG. 10 shows the results of comparing the presence or absence of prokFlaB- or eukFlaB-selective antibody production when *Vibrio vulnificus-derived* wild-type flagellin (prokFlaB) expressed in *E. coli* cells, which are prokaryotic cells, wild-type flagellin (eukFlaB) expressed in Expi293^{™} cells, which are eukaryotic cells, eukFlaB^{N83/101/139A}, eukFlaB^{N83/101/139/273A}, and eukFlaB^{N83/101/139/273/330A} were used as adjuvants in the influenza vaccine model according to an example of the present invention.

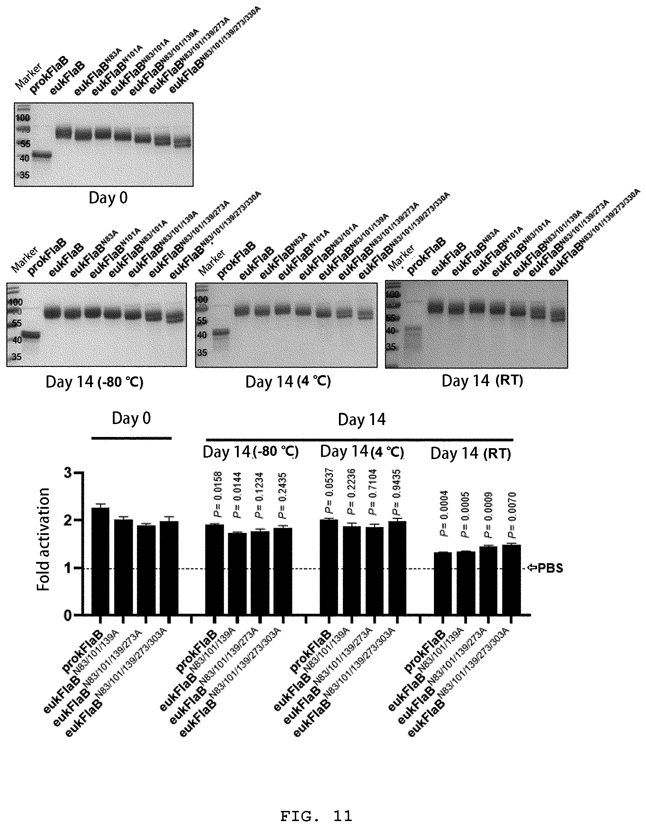

FIG. 11 shows the results of comparing stability among *Vibrio vulnificus-derived* wild-type flagellin (prokFlaB) expressed in *E. coli* cells, which are prokaryotic cells, wild-type flagellin (eukFlaB) expressed in Expi293^{™} cells, which are eukaryotic cells, and various flagellin variants in the influenza vaccine model according to an example of the present invention.

### Best Mode for Carrying out the Invention

The present invention relates to a polypeptide expressible in a eukaryotic cell, in which at least one asparagine (N) residue in the amino acid sequence of SEQ ID NO: 13 is substituted with alanine.

### Mode for Carrying out the Invention

Hereinafter, the present invention will be described in more detail by the following exemplary embodiments. However, these exemplary embodiments are used only for illustration, and the scope of the present invention is not limited by these exemplary embodiments.

Throughout the present specification, the "%" used to express the concentration of a specific material, unless otherwise particularly stated, refers to (wt/wt)% for solid/solid, (wt/vol)% for solid/liquid, and (vol/vol)% for liquid/liquid.

### Example 1: Construction of plasmid for production of eukaryotic-expressed wild-type flagellin

*Vibrio vulnificus-derived* FlaB (prokFlaB) expressed in eukaryotic cells was produced by the method disclosed in Lee SE, et al. A bacterial flagellin, Vibrio vulnificus FlaB, has a strong mucosal adjuvant activity to induce protective immunity. Infect Immun. 2006;74(1):694-702.

To express FlaB in eukaryotic cells, the amplification was performed through PCR using the forward primer eukFlaB-F containing a *Hind*III (AAGCTT) cleavage site and the reverse primer eukFlaB-R containing a *NotI* cleavage site, as shown in Table 1 below. Specifically, pBHA: :eukFlaB (Bioneer, South Korea), which was obtained by synthesizing FlaB, optimized according to the codon usage (frequency) for expression in mammalian cells, and then cloning the FlaB into the pBHA plasmid, was used as a template for PCR.

**TABLE 1**

| SEQ ID NO | Name | Sequence (5'->3') |
|---|---|---|
| 1 | eukFlaB-F | CCCAAGCTTGCCGTCAACGTGAACACCAA |
| 2 | eukFlaB-R | ATAGTTTAGCGGCCGCGCCCAGCAGGGAGAGGGC GC |

The eukFlaB DNA amplification product and the eukaryotic-expressed vector pSectag2B (Invitrogen, V900-20) were separately treated with *HindI*II and NotI, a pair of restriction enzymes (RE), at 37°C overnight. The PCR product and the eukaryotic-expressed vector pSectag2B, which had been treated with restriction enzymes, were ligated by T4 DNA ligase (Enzynomics) at room temperature for 2 hours. The new structure pSectag2B::eukFlaB plasmid was transformed into the *E. coli* competent cells TOP10 (Invitrogen), and cultured on LB plates containing ampicillin. The DNA sequence of the expression vector was confirmed by dideoxy-chain termination sequencing through the Macrogen Online Sequencing Order System (http://dna. macrogen.com/kor/) .

### Example 2: Production of recombinant protein utilizing eukaryotic expression system

The eukaryotic expression cell line, Expi293^{™} cells, was purchased from Thermo Fisher Scientific (Cat#. A14635) and used in the present invention. Transfection was performed according to the manufacturer's protocol. The Expi293^{™} cells were cultured at 37°C, 8% CO₂, and 125 rpm shaking speed of incubator. On Day 0 (the day before transfection), the Expi293^{™} cells were seeded at 3×10⁶ cells/mL in culture flasks (Corning). On Day 1 (the day of transfection), the Expi293^{™} cells counted to 3x10⁶/mL were seeded, and the pSectag2B::eukFlaB plasmid sterilized by filtration through a 0.22-µm filter (Millex-GV, SLGVR13) were added to the cell culture system. On day 2, ExpiFectamine^{™} 293 Transfection Enhancer 1 and Enhancer 2 were added and cultured until Day 4. Specifically, the recombinant protein was secreted into the culture medium due to pSectag2B containing the IgK chain sequence.

On day 4, the culture medium containing the secreted protein was centrifuged at 17,000 rpm at 4°C for 20 minutes, and then the cell-free supernatant was loaded onto a column containing Ni-NTA agarose bead (Qiagen, Hilden, Germany) According to the manufacturer's instructions. To remove proteins other than the desired protein, the column was washed with washing buffer I (50 mM NaH₂PO₄, 300 mM NaCl, 10 mM Imidazole, pH=8) and washing buffer II (50 mM NaH₂PO₄, 300 mM NaCl, and 20 mM Imidazole, pH=8). The target protein was eluted and separated using an elution buffer (50 mM NaH₂PO₄, 300 mM NaCl and 250 mM Imidazole, pH=8) at room temperature for 15 minutes. The proteins thus eluted were purified by dialysis, and the buffer was changed to phosphate-buffered saline (PBS).

The recombinant peptide was confirmed by SDS-PAGE and Western blot analysis using anti-FlaB antibody generated in BALB/c mice, and the results are shown in FIG. 1.

### Example 3: Verification of TLR5 stimulating activity of wild-type flagellin constructed using eukaryotic expression system

To verify TLR5 stimulating activity of wild-type flagellin constructed utilizing a eukaryotic expression system, the activity of NF-κB at various protein concentrations was measured. Specifically, HEK-Blue^{™} hTLR5 cells (InvivoGen, hκb-htlr-5) with HEK-Blue^{™} detection (InvivoGen, hb-det2) assay systems were used according to the manufacturer's protocol. The EC50 for each concentration of protein was calculated using triplicate OD 620 nm values. To determine the biological activity of deglycosylated eukFlaB, in vitro NF-κB luciferase reporter assay was performed using HEK293T cells purchased from Thermo fisher Scientific. HEK293T cells were maintained in DMEM medium (Gibco^{™}, cat.11995-065) containing 10% FBS, 100 units/ml of an antibiotic (Gibco^{™}, cat.15140122) at 37°C and 5% CO₂. At least 3 passages of cells were used for the NF-κB reporter assay. HEK293T cells were seeded at 2x10⁵/well in 24-well plates (SPL Life Sciences Co., Ltd., Korea) and incubated for 24 hours. For transfection, the cells were incubated with the reporter plasmid pNF-κB-luc (100 ng/well), p3xFlag-hTLR5 (100 ng/well), pCMV-β-Gal (50 ng/well), and Effectene (5 *µ*ℓ/well) (Qiagen, 301427) overnight. The transfected cells were treated with prokFlaB, eukFlaB, or mutated eukFlaB and incubated for 24 hours. Then, the media were removed, and the cells were lysed with a cell lysis buffer (Promega Madison, WI USA, E153A). The luciferase activities in the cell lysates were measured using a luminometer (Berthold, Lumat-Plus LB96V).

As a result, as shown in FIG. 2, eukFlaB induced a significantly lower TLR5 stimulation compared with prokFlaB.

### Example 4: Verification of N-deglycosylation of prokaryotic-expressed flagellin using deglycosylase (Peptide N-glycosidase F) and TLR5 stimulating activity of deglycosylated prokaryotic-expressed flagellin

The prokaryotic-expressed eukFlaB were deglycosylated by PNGase F enzyme (Promega, cat.V483A) according to the manufacturer's protocol. PNGase F enzyme (10 U/µl) and eukFlaB (15 µg) were mixed and incubated at 37°C. After 2 hours, the degree of deglycosylation was examined by SDS-PAGE and Western blot analysis using anti-FlaB antibody generated in BALB/c mice, and the results are shown in FIG. 3A. The TLR5 stimulating activity of the deglycosylated eukFlaB was determined by the same method as in Example 3, and the results are shown in FIG. 3B.

As a result, as shown in FIG. 3B, the deglycosylated eukFlaB restored TLR5 stimulating activity.

### Example 5: Fabrication of various deglycosylated flagellin derivatives through site-directed mutagenesis and verification of TLR5 stimulating activities of various deglycosylated eukaryotic-expressed flagellin derivatives

The N-glycosylation sites of the FlaB amino acid sequence were predicted using NetNGlyc-1.0 (https://services.healthtech.dtu.dk/service.php?NetNGlyc -1.0), a bioinformatic tool that determines the N-glycosylation consensus sequence Asn-Xaa-Ser/Thr.

As shown in FIG. 4, as for eukFlaB, five putative glycosylating residues were predicted at N83, N101, N139, N273, and N330, and three residues (N83, N101, N139) were identified to be located near the TLR5 binding domain.

Therefore, as shown in FIG. 5, site-directed mutagenesis was induced to construct various deglycosylated flagellin proteins [eukFlaB^{N83A}, eukFlaB^{N101A}, eukFlaB^{N139A}, eukFlaB^{N273A}, eukFlaB^{N330A}, eukFlaB^{N83A,N101A} (eukFlaB^{N83/101A}), eukFlaB^{N83A,N101A,N139A} (eukFlaB^{N83/101/139A}), eukFlaB^{N83A,N101A,N139A,N273A} (eukFlaB^{N83/101/139/273A}), eukFlaB^{N83A,N101A,N139A,N273A,N330A} (eukFlaB^{N83/101/139/273/330A}), and eukFlaB^{N139A,N273A,N330A} (eukFlaB^{N139/273/330A})].

Site-directed mutation of eukFlaB was conducted according to the manufacturer's protocol (EZchange^{™} Site-directed Mutation, Cat. # EZ004S). Briefly, pSectag2B::eukFlaB plasmid was used as a template for PCR reaction, and especially, the used primers in Table 2 were designed for site-directed mutation where the amino acid asparagine (N) corresponding to the specific codon "AAC" or "AAT" was changed into another specific codon amino acid alanine (A) corresponding to "GCC" or "GCT". After the parental plasmid was separated from the PCR product using EZ-MIX enzyme, the plasmid was transformed into *E*. *coli* competent cells (TOP10), and the transformed cells were evenly spread onto LB plates containing ampicillin (100 µg/ml). The positively mutated eukFlaB sequences were confirmed by Macrogen Online Sequencing System (http://dna.macrogen.com/kor/).

**TABLE 2**

| SEQ ID NO | Name | Sequence (5'->3') |
|---|---|---|
| 3 | N83A-F | CTGAAGGCGCCATGGCCGAAACCACCAACATC |
| 4 | N83A-R | GATGTTGGTGGTTTCGGCCATGGCGCCTTCAG |
| 5 | N101A-F | CTCTGCAGTCTGCCGCCGGCAGCAACAGCAAG |
| 6 | N101A-R | CTTGCTGTTGCTGCCGGCGGCAGACTGCAGAG |
| 7 | N139A-F | CAACAAGCTGCTGGCTGGCACCTACGGCAC |
| 8 | N139A-R | GTGCCGTAGGTGCCAGCCAGCAGCTTGTTG |
| 9 | N273A-F | GGGCGAGGGCAAGGCCGTGACCGTGGATAC |
| 10 | N273A-R | GTATCCACGGTCACGGCCTTGCCCTCGCCC |
| 11 | N330A-F | CAACGAGAACGTCGCTGCCTCTAAGTCCAGG |
| 12 | N330A-R | CCTGGACTTAGAGGCAGCGACGTTCTCGTTG |

The recombinant peptides were confirmed by SDS-PAGE and Western blot analysis using anti-FlaB antibody generated in BALB/c mice, and the results are shown in FIG. 6.

The peptide sequences of eukFlaB, eukFlaB^{N83A}, eukFlaB^{N101A}, eukFlaB^{N139A}, eukFlaB^{N273A}, eukFlaB^{N330A}, eukFlaB^{N83/101A}, eukFlaB^{N83/101/139A}, eukFlaB^{N83/101/139/273A}, eukFlaB^{N83/101/139/273/330A}, and eukFlaB^{N139/273/330A} manufactured by the above-described method are shown in Table 3.

**TABLE 3**

| SEQ ID NO | Name | Sequence (5'->3') |
|---|---|---|
| 13 | eukFlaB | MAVNVNTNVAAMTAQRYLNNANSAQQTSMERLS SGFKINSAKDDAAGLQISNRLNVQSRGLDVAVR NANDGISIAQTAEGAMNETTNILQRMRDLSLQS ANGSNSKSERVAIQEEVTALNDELNRIAETTSF GGNKLLNGTYGTKAMQIGADNGEAVMLSLKDMR SDNVMMGGVSYQAEEGKDKNWNVAAGDNDLTIA LTDSFGNEQEIEINAKAGDDIEELATYINGQTD LVKASVGEGGKLQIFAGNNKVQGEIAFSGSLAG ELGLGEGKNVTVDTIDVTTVQGAQESVAIVDAA LKYVDSHRAELGAFQNRFNHAISNLDNINENVN ASKSRIKDTDFAKETTQLTKTQILSQASSSILA QAKQAPNSALSLLG |
| 14 | eukFlaB^{N83A} | MAVNVNTNVAAMTAQRYLNNANSAQQTSMERLS SGFKINSAKDDAAGLQISNRLNVQSRGLDVAVR NANDGISIAQTAEGAMAETTNILQRMRDLSLQS ANGSNSKSERVAIQEEVTALNDELNRIAETTSF GGNKLLNGTYGTKAMQIGADNGEAVMLSLKDMR SDNVMMGGVSYQAEEGKDKNWNVAAGDNDLTIA LTDSFGNEQEIEINAKAGDDIEELATYINGQTD LVKASVGEGGKLQIFAGNNKVQGEIAFSGSLAG ELGLGEGKNVTVDTIDVTTVQGAQESVAIVDAA LKYVDSHRAELGAFQNRFNHAISNLDNINENVN ASKSRIKDTDFAKETTQLTKTQILSQASSSILA QAKQAPNSALSLLG |
| 15 | eukFlaB^{N101 A} | MAVNVNTNVAAMTAQRYLNNANSAQQTSMERLS SGFKINSAKDDAAGLQISNRLNVQSRGLDVAVR NANDGISIAQTAEGAMNETTNILQRMRDLSLQS |
| | | AAGSNSKSERVAIQEEVTALNDELNRIAETTSF GGNKLLNGTYGTKAMQIGADNGEAVMLSLKDMR SDNVMMGGVSYQAEEGKDKNWNVAAGDNDLTIA LTDSFGNEQEIEINAKAGDDIEELATYINGQTD LVKASVGEGGKLQIFAGNNKVQGEIAFSGSLAG ELGLGEGKNVTVDTIDVTTVQGAQESVAIVDAA LKYVDSHRAELGAFQNRFNHAISNLDNINENVN ASKSRIKDTDFAKETTQLTKTQILSQASSSILA QAKQAPNSALSLLG |
| 16 | eukFlaB^{N139} A | MAVNVNTNVAAMTAQRYLNNANSAQQTSMERLS SGFKINSAKDDAAGLQISNRLNVQSRGLDVAVR NANDGISIAQTAEGAMNETTNILQRMRDLSLQS ANGSNSKSERVAIQEEVTALNDELNRIAETTSF GGNKLLAGTYGTKAMQIGADNGEAVMLSLKDMR SDNVMMGGVSYQAEEGKDKNWNVAAGDNDLTIA LTDSFGNEQEIEINAKAGDDIEELATYINGQTD LVKASVGEGGKLQIFAGNNKVQGEIAFSGSLAG ELGLGEGKNVTVDTIDVTTVQGAQESVAIVDAA LKYVDSHRAELGAFQNRFNHAISNLDNINENVN ASKSRIKDTDFAKETTQLTKTQILSQASSSILA QAKQAPNSALSLLG |
| 17 | eukFlaB^{N273} A | MAVNVNTNVAAMTAQRYLNNANSAQQTSMERLS SGFKINSAKDDAAGLQISNRLNVQSRGLDVAVR NANDGISIAQTAEGAMNETTNILQRMRDLSLQS ANGSNSKSERVAIQEEVTALNDELNRIAETTSF GGNKLLNGTYGTKAMQIGADNGEAVMLSLKDMR SDNVMMGGVSYQAEEGKDKNWNVAAGDNDLTIA LTDSFGNEQEIEINAKAGDDIEELATYINGQTD LVKASVGEGGKLQIFAGNNKVQGEIAFSGSLAG ELGLGEGKAVTVDTIDVTTVQGAQESVAIVDAA LKYVDSHRAELGAFQNRFNHAISNLDNINENVN ASKSRIKDTDFAKETTQLTKTQILSQASSSILA QAKQAPNSALSLLG |
| 18 | eukFlaB^{N330 A} | MAVNVNTNVAAMTAQRYLNNANSAQQTSMERLS SGFKINSAKDDAAGLQISNRLNVQSRGLDVAVR NANDGISIAQTAEGAMNETTNILQRMRDLSLQS ANGSNSKSERVAIQEEVTALNDELNRIAETTSF GGNKLLNGTYGTKAMQIGADNGEAVMLSLKDMR SDNVMMGGVSYQAEEGKDKNWNVAAGDNDLTIA LTDSFGNEQEIEINAKAGDDIEELATYINGQTD LVKASVGEGGKLQIFAGNNKVQGEIAFSGSLAG ELGLGEGKNVTVDTIDVTTVQGAQESVAIVDAA LKYVDSHRAELGAFQNRFNHAISNLDNINENVA ASKSRIKDTDFAKETTQLTKTQILSQASSSILA QAKQAPNSALSLLG |
| 19 | ^{e}ukFlaB^{N83/ 101A} | MAVNVNTNVAAMTAQRYLNNANSAQQTSMERLS SGFKINSAKDDAAGLQISNRLNVQSRGLDVAVR |
| | | NANDGISIAQTAEGAMAETTNILQRMRDLSLQS AAGSNSKSERVAIQEEVTALNDELNRIAETTSF GGNKLLNGTYGTKAMQIGADNGEAVMLSLKDMR SDNVMMGGVSYQAEEGKDKNWNVAAGDNDLTIA LTDSFGNEQEIEINAKAGDDIEELATYINGQTD LVKASVGEGGKLQIFAGNNKVQGEIAFSGSLAG ELGLGEGKNVTVDTIDVTTVQGAQESVAIVDAA LKYVDSHRAELGAFQNRFNHAISNLDNINENVN ASKSRIKDTDFAKETTQLTKTQILSQASSSILA QAKQAPNSALSLLG |
| 20 | eukFlaB^{N83/ 101/139A} | MAVNVNTNVAAMTAQRYLNNANSAQQTSMERLS SGFKINSAKDDAAGLQISNRLNVQSRGLDVAVR NANDGISIAQTAEGAMAETTNILQRMRDLSLQS AAGSNSKSERVAIQEEVTALNDELNRIAETTSF GGNKLLAGTYGTKAMQIGADNGEAVMLSLKDMR SDNVMMGGVSYQAEEGKDKNWNVAAGDNDLTIA LTDSFGNEQEIEINAKAGDDIEELATYINGQTD LVKASVGEGGKLQIFAGNNKVQGEIAFSGSLAG ELGLGEGKNVTVDTIDVTTVQGAQESVAIVDAA LKYVDSHRAELGAFQNRFNHAISNLDNINENVN ASKSRIKDTDFAKETTQLTKTQILSQASSSILA QAKQAPNSALSLLG |
| 21 | eukFlaB^{N83/ 101/139/273A} | MAVNVNTNVAAMTAQRYLNNANSAQQTSMERLS SGFKINSAKDDAAGLQISNRLNVQSRGLDVAVR NANDGISIAQTAEGAMAETTNILQRMRDLSLQS AAGSNSKSERVAIQEEVTALNDELNRIAETTSF GGNKLLAGTYGTKAMQIGADNGEAVMLSLKDMR SDNVMMGGVSYQAEEGKDKNWNVAAGDNDLTIA LTDSFGNEQEIEINAKAGDDIEELATYINGQTD LVKASVGEGGKLQIFAGNNKVQGEIAFSGSLAG ELGLGEGKAVTVDTIDVTTVQGAQESVAIVDAA LKYVDSHRAELGAFQNRFNHAISNLDNINENVN ASKSRIKDTDFAKETTQLTKTQILSQASSSILA QAKQAPNSALSLLG |
| 22 | eukFlaB^{N83/ 101/139/273/330 A} | MAVNVNTNVAAMTAQRYLNNANSAQQTSMERLS SGFKINSAKDDAAGLQISNRLNVQSRGLDVAVR NANDGISIAQTAEGAMAETTNILQRMRDLSLQS AAGSNSKSERVAIQEEVTALNDELNRIAETTSF GGNKLLAGTYGTKAMQIGADNGEAVMLSLKDMR SDNVMMGGVSYQAEEGKDKNWNVAAGDNDLTIA LTDSFGNEQEIEINAKAGDDIEELATYINGQTD LVKASVGEGGKLQIFAGNNKVQGEIAFSGSLAG ELGLGEGKAVTVDTIDVTTVQGAQESVAIVDAA LKYVDSHRAELGAFQNRFNHAISNLDNINENVA ASKSRIKDTDFAKETTQLTKTQILSQASSSILA QAKQAPNSALSLLG |
| 23 | eukFlaB^{N139/273/330A} | MAVNVNTNVAAMTAQRYLNNANSAQQTSMERLS SGFKINSAKDDAAGLQISNRLNVQSRGLDVAVR NANDGISIAQTAEGAMNETTNILQRMRDLSLQS ANGSNSKSERVAIQEEVTALNDELNRIAETTSF GGNKLLAGTYGTKAMQIGADNGEAVMLSLKDMR SDNVMMGGVSYQAEEGKDKNWNVAAGDNDLTIA LTDSFGNEQEIEINAKAGDDIEELATYINGQTD LVKASVGEGGKLQIFAGNNKVQGEIAFSGSLAG ELGLGEGKAVTVDTIDVTTVQGAQESVAIVDAA LKYVDSHRAELGAFQNRFNHAISNLDNINENVA ASKSRIKDTDFAKETTQLTKTQILSQASSSILA QAKQAPNSALSLLG |

To determine the biological activity of various flagellin variants, the activity of NF-κB mediated by Toll-like receptor 5-signaling was measured by reporter assay. HEK293T cells were maintained in DMEM medium (Gibco^{™}, cat.11995-065) containing 10% FBS (Gibco^{™}, cat.16000044), 100 units/ml of penicillin, 100 *µ*g/ml of the antibiotic streptomycin (Gibco^{™}, cat.15140122). HEK293T cells were seeded at 2x10⁵/well in 24-well plates (Corning costar, cat.3526) and incubated at 37°C and 5% CO₂ in an incubator for 24 hours. For transfection, the cells were incubated with the reporter plasmid pNF-κB-luc (100 ng/well), p3xFlag-hTLR5 (100 ng/well), pCMV-β-Gal (50 ng/well) Effectene (5 µℓ/well) (Qiagen, 301427) overnight. The transfected cells were treated with prokFlaB, eukFlaB, or mutated eukFlaB and incubated for 24 hours. Then, the media were removed, and the cells were lysed with a cell lysis buffer. Thereafter, the luciferase activities in the cell lysates were measured using a luminometer (Berthold, Lumat-Plus LB96V) to verify TLR5 signaling activities.

As shown in FIG. 7, the test results confirmed that eukFlaB, eukFlaB^{N101A}, eukFlaB^{N139A}, eukFlaB^{N273A}, eukFlaB^{N330A}, eukFlaB^{N139/273/330A} did not induce TLR5 activation, but eukFlaB^{N83A}, eukFlaB^{N83/101a}, eukFlaB^{N83/101/139A}, eukFlaB^{N83/101/139/273A}, and eukFlaB^{N83/101/139/273/330A} induced TLR5 activation. Especially, eukFlaB^{N83/101/139A}, eukFlaB^{N83/101/139/273A}, and eukFlaB^{N83/101/139/273/330A} had TLR5 stimulating activity equivalent to that of prokaryotic cell-derived flagellin (prokFlaB).

### Example 6: Determination of TLR5 binding with flagellin variants using co-immunoprecipitation (Co-IP)

As follows, the binding of TLR5 with flagellin variants was measured using co-immunoprecipitation (Co-IP). Specifically, HEK293T cells were transfected with p3XFlag-hTLR5 plasmid for 24 hours. The cells were lysed using RIPA buffer containing protease inhibitors, sonicated, and then centrifuged to collect the supernatant. The proteins, prokFlaB, eukFlaB, eukFlaBN83/101/139A, and eukFlaBN83/101/139/273/330A were mixed with the supernatant, followed by rotating at 4°C overnight (Complex I). At the same time, the Protein A/G plusagarose (Santa Cruz Biotechnology, sc-2003) was mixed with an anti-Flag tag antibody (Abcam, ab1162), followed by rotation at 4°C overnight (complex II). Then, Complex II was washed with IP buffer (1% TritonX-100, 25 mM Tris pH 7.5, 10% Glycerol, 150 mM NaCl, 1 mM DTT, 1 mM EDTA, and protease inhibitor) five times. Complex I and Complex II were mixed at 4°C for 6 hours, and then washed with IP buffer five times to remove the nonspecific binding proteins. After 50 µl of 1x SDS loading buffer was added and heated for 10 minutes, SDS-PAGE and further western blot were analyzed. Two different antibodies, mouse anti-FlaB and anti-Myc-HRP (Novex^{®}, 46-0709) were used for Western blot assay to detect pFlaB and eukFlaB variants, respectively, because the detected eukFlaB proteins were low due to glycosylation on eukFlaB Since anti-FlaB eukFlaB variants were expressed along with Myc-tag, which was well detected by anti-Myc.

As shown in FIG. 8, the test results confirmed that prokFlaB bound to TLR5 with high affinity but did not bind to eukFlaBsms TLR5. It was also confirmed that the eukFlaBN83/101/139A and eukFlaBN83/101/139/273/330A variants restored TLR5 binding. These results confirmed that glycosylation interfered with the FlaB-TLR5 binding and deglycosylation resulted in higher affinity of FlaB-TLR5 binding.

### Example 7: Verification of adjuvant activity of constructed eukaryotic-expressed flagellin variants in influenza vaccine test model system utilizing mice

Seven-week-old female BALB/C mice (n=10) were purchased from ORIENT (Seongnam-si, Gyeonggi-do, Korea) and acclimated to the facility environment for one week before experiments. The mice were anesthetized by intraperitoneal administration with a blend of Zoletil^{®} 50 (Virbac corporation, Carros, France) and Rompun^{™} (Bayer AG, Leverkusen, Germany) prior to immunization. Intranasal immunization was started using groups: 1) sH1N1 antigen (sH1N1, 0.2 ug/mouse), 2) mixture of sH1N1 (0.2 ug/mouse) with prokFlaB (4 ug/mouse), 3) or with eukFlaB (4 ug/mouse), (sH1N1+prokFlaB, sH1N1+eukFlaB, sH1N1+eukFlaB^{N83A}, sH1N1+eukFlaB^{N83/101A}, sH1N1+eukFlaB^{N83/101/139A}, sH1N1+eukFlaB^{N83/101/139/273A.}, and sH1N1+eukFlaB^{N83/101/139/273/330A}). The vaccine antigens were diluted in PBS and used at 10 µL per nostril, for a total volume of 20 µl/mouse. The mice were immunized three times at 2-week intervals, and after 2 weeks of final immunization, the serum was taken for assay.

The influenza antigen-specific antibody immune responses were measured by enzyme-linked immunosorbent assay (ELISA), and the mouse serum was collected two weeks after the third immunization. ELISA plates (Corning Laboratories, 3690) were coated with sH1N1 (4 µg/ml), and then fixed at 4°C overnight. Next day, the plates were washed three times with the washing buffer PBS-T [PBS containing 0.05% of Tween20 (Thermo fisher scientific, cat. J20605-AP)], and blocked with a blocking buffer [1 mM of EDTA (JUNSEI, cat. 17385S0401) and 0.5% of BSA (Sigma, cat. A2153-50G) in PBS-T] at room temperature for one hour, thereby blocking antigen and antibody nonspecific binding. The serum was serially diluted (2-fold dilution) in a blocking buffer and added as 40 µl/well, followed by incubation at room temperature for 2 hours. The plates were washed five times with PBS-T, and the horseradishperoxidase(HRP) conjugated anti-mouse IgG antibody was added, followed by incubation at room temperature for 1 hour. The plates were washed with PBS-T. Thereafter, the reaction was stopped by addition of the STOP buffer (1 N H₂SO₄) when the signal was developed by adding the substrate TMB (BD OptEIA, cat.555214). The absorbance was measured at 450 nm by using a microplate reader (Molecular Devices Corporation, Menlo Park, CA). The antibody titer was computed as the reciprocal Log2 value of the serum dilution which yielded 2-fold higher values of absorbance than the blank well.

As shown in FIG. 9, the test results confirmed that eukFlaB^{N83A}, eukFlaB^{N83/101A}, eukFlaB^{N83/101/139A}, eukFlaB^{N83/101/139/273A}, and eukFlaB^{N83/101/139/273/330A} had adjuvant efficacy by inducing the production of influenza antigen-specific antibodies at the same level as in prokaryotic-expressed flagellin (prokFlaB).

### Example 8: Verification of comparison of antibody production ability between prokaryotic-expressed flagellin and eukaryotic-expressed flagellin

In the influenza vaccine model of Example 7, flagellin-specific antibody immune responses were measured by enzyme-linked immunosorbent assay (ELISA). Specifically, the mice serum was collected two weeks after the third immunization. ELISA plates (Corning Laboratories, 3690) were coated with prokFlaB (1 *µ*g/ml) or wild-type eukFlaB (1 *µ*g/ml) and then fixed at 4°C overnight. Enzyme-linked immunosorbent assay (ELISA) was conducted by the same method as in Example 7.

As shown in FIG. 10, the test results confirmed that the prokaryotic-expressed flagellin (prokFlaB) induced the production of prokFlaB- and eukFlaB-specific antibodies, but eukFlaB, eukFlaB^{N83/101/139A}, eukFlaB^{N83/101/139/273A}, and eukFlaB^{N83/101/139/273/330A} did not induce the production of prokFlaB- and eukFlaB-specific antibodies. It was therefore identified that the eukFlaB variants of the present invention did not induce immune responses for flagellin itself, which is a biological agent, by repeated administration in vivo, and thus can provide safer flagellin adjuvant.

### Example 9: Verification of safety of deglycosylated prokaryotic-expressed flagellin derivatives

After purification, prokFlaB, eukFlaB, and N-glycosylation mutant eukFlaB were stored at -80°C, 4°C, and a temperature different from room temperature for two weeks. The protein structural stability and functionality were tested by sodium dodecyl sulfate-polyacrylamide gel electrophoresis (SDS-PAGE) and NF -κB reporter assay, respectively.

As shown in FIG. 11, the test results confirmed that eukaryotic-expressed flagellin had superior stability compared with prokaryotic-expressed flagellin (prokFlaB). It was therefore predicted that the eukFlaB variants of the present invention can provide pharmacokinetically superior adjuvants in the production, distribution, and in vivo administration of biological agents.

### Industrial Applicability

The present invention relates to the manufacture and application of a flagellin adjuvant derivative with TLR5 activity using a eukaryotic cell expression system and, more specifically, to a flagellin protein that can be expressed in a eukaryotic cell and exhibit an immunopotentiating effect equivalent to or higher than that of an existing prokaryote-derived flagellin protein.

## Claims

1. A polypeptide expressible in a eukaryotic cell, in which at least one asparagine (N) residue in the amino acid sequence of SEQ ID NO: 13 is substituted with alanine.

2. The polypeptide of claim 1, wherein an asparagine residue at at least one position selected from the group consisting of positions 83, 101, 139, 273, and 330 in the amino acid sequence of SEQ ID NO: 13 is substituted with alanine.

3. The polypeptide of claim 1, wherein the polypeptide includes one amino acid sequence selected from the group consisting of amino acid sequences of SEQ ID NOS: 14 to 23.

4. The polypeptide of claim 2, wherein asparagine residues at position 83 and at least one position selected from the group consisting of positions 101, 139, 273, and 330 in the amino acid sequence of SEQ ID NO: 13 are substituted with alanine.

5. The polypeptide of claim 2, wherein asparagine residues at positions 83 and 101 and at least one position selected from the group consisting of positions 139, 273, and 330 in the amino acid sequence of SEQ ID NO: 13 are substituted with alanine.

6. The polypeptide of claim 2, wherein asparagine residues at positions 83, 101, and 139 and at least one position selected from the group consisting of positions 273 and 330 in the amino acid sequence of SEQ ID NO: 13 are substituted with alanine.

7. An adjuvant composition for a vaccine, comprising a polypeptide in which at least one asparagine (N) residue is substituted with alanine in the amino acid sequence of SEQ ID NO: 13.

8. The adjuvant composition of claim 7, wherein in the polypeptide, an asparagine residue at at least one position selected from the group consisting of positions 83, 101, 139, 273, and 330 in the amino acid sequence of SEQ ID NO: 13 is substituted with alanine.

9. The adjuvant composition of claim 7, wherein the polypeptide includes one amino acid sequence selected from the group consisting of amino acid sequences of SEQ ID NOS: 14 to 23.

10. The adjuvant composition of claim 8, wherein in the polypeptide, asparagine residues at position 83 and at least one position selected from the group consisting of positions 101, 139, 273, and 330 in the amino acid sequence of SEQ ID NO: 13 are substituted with alanine.

11. The adjuvant composition of claim 8, wherein in the polypeptide, asparagine residues at positions 83 and 101 and at least one position selected from the group consisting of positions 139, 273, and 330 in the amino acid sequence of SEQ ID NO: 13 are substituted with alanine.

12. The adjuvant composition of claim 8, wherein in the polypeptide, asparagine residues at positions 83, 101, and 139 and at least one position selected from the group consisting of positions 273 and 330 in the amino acid sequence of SEQ ID NO: 13 are substituted with alanine.

13. A composition for immune function enhancement, the composition comprising a polypeptide in which at least one asparagine (N) residue is substituted with alanine in the amino acid sequence of SEQ ID NO: 13.

14. The composition of claim 13, wherein in the polypeptide, an asparagine residue at at least one position selected from the group consisting of positions 83, 101, 139, 273, and 330 in the amino acid sequence of SEQ ID NO: 13 is substituted with alanine.

15. The composition of claim 13, wherein the polypeptide includes one amino acid sequence selected from the group consisting of amino acid sequences of SEQ ID NOS: 14 to 23.

16. The composition of claim 14, wherein in the polypeptide, asparagine residues at position 83 and at least one position selected from the group consisting of positions 101, 139, 273, and 330 in the amino acid sequence of SEQ ID NO: 13 are substituted with alanine.

17. The composition of claim 14, wherein in the polypeptide, asparagine residues at positions 83 and 101 and at least one position selected from the group consisting of positions 139, 273, and 330 in the amino acid sequence of SEQ ID NO: 13 are substituted with alanine.

18. The adjuvant composition of claim 14, wherein in the polypeptide, asparagine residues at positions 83, 101, and 139 and at least one position selected from the group consisting of positions 273 and 330 in the amino acid sequence of SEQ ID NO: 13 are substituted with alanine.

19. A pharmaceutical composition for preventing or treating an autoimmune disease, an inflammatory disease, a microbial infection, or cancer, the pharmaceutical composition comprising a polypeptide in which at least one asparagine (N) residue is substituted with alanine in the amino acid sequence of SEQ ID NO: 13.

20. The pharmaceutical composition of claim 19, wherein in the polypeptide, an asparagine residue at at least one position selected from the group consisting of positions 83, 101, 139, 273, and 330 in the amino acid sequence of SEQ ID NO: 13 is substituted with alanine.

21. The pharmaceutical composition of claim 19, wherein the polypeptide includes one amino acid sequence selected from the group consisting of amino acid sequences of SEQ ID NOS: 14 to 23.

22. The pharmaceutical composition of claim 20, wherein in the polypeptide, asparagine residues at position 83 and at least one position selected from the group consisting of positions 101, 139, 273, and 330 in the amino acid sequence of SEQ ID NO: 13 are substituted with alanine.

23. The pharmaceutical composition of claim 20, wherein in the polypeptide, asparagine residues at positions 83 and 101 and at least one position selected from the group consisting of positions 139, 273, and 330 in the amino acid sequence of SEQ ID NO: 13 are substituted with alanine.

24. The pharmaceutical composition of claim 20, wherein in the polypeptide, asparagine residues at positions 83, 101, and 139 and at least one position selected from the group consisting of positions 273 and 330 in the amino acid sequence of SEQ ID NO: 13 are substituted with alanine.
